# EUROPEAN PATENT SPECIFICATION

(11) **EP 0 911 330 B1**
(45) Date of publication and mention of the grant of the patent: **12.12.2007**
(21) Application number: 97308391.8
(22) Date of filing: 22.10.1997
(51) Int. Cl.: C07D 403/06, A61K 31/495

(54) **1-(4-Arylpiperazine-1-y1)-3-(2-oxopyrrolidin/piperidin-1-y1) propanes as therapeutic agents for hypertension, ischemia, cardiovascular and other adrenergic receptors related disorders**
1-(4-Arylpiperazin-1-yl)-3-(2-oxopyrrolidin/piperidin-1-yl)propane als therapeutische Mittel bei Bluthochdruck, Ischämie,kardiovaskulären und anderen,mit adrenergenen Rezeptoren im Zusammenhang stehenden Störungen
1-(4-Arylpipérazin-1-yl)-3-(2-oxopyrrolidin/pipéridin-1-yl)propanes comme agents thérapeutiques pour l'hypertension, l'ischémie, des troubles cardiovasculaires et d'autres troubles liés aux récepteurs adrénergiques

(43) Date of publication of application: 28.04.1999
(73) Proprietor: Council of Scientific & Industrial Research, New Delhi 110001 (IN)
(72) Inventor: Sinha, Neelima, Lucknow 226 001, U.P. (IN); Jain, Sanjay, Lucknow 226 001, U.P. (IN); Saxena, Anil Kumar, Lucknow 226 001, U.P. (IN); Anand, Nitya, Lucknow 226 001, U.P. (IN); Saxena, Ram Mohan, Lucknow 226 001, U.P. (IN); Dubey, Mangal Prasad, Lucknow 226 001, U.P. (IN); Patnaik, Gyanendra Kumar, Lucknow 226 001, U.P. (IN)
(74) Representative: Wain, Christopher Paul

(56) References cited:
- GB-A- 2 023 594
- CHEMICAL ABSTRACTS, vol. 100, no. 7, 13 February 1984 Columbus, Ohio, US; abstract no. 44907p, B. MALAWSKA ET AL.: "Synthesis and pharmacological properties of some 2-pyrrolidinone Mannich bases" page 17; XP002056654 & POL. J. PHARMACOL. PHARM., vol. 34, no. 5-6, 1982, pages 373-382,
- P. E. CROSS ET AL.: "Substituted trifluoromethyl phenyl piperazines as anorectic agents" EUR. J. MED. CHEM. - CHIM. THER., vol. 12, no. 2, 1977, pages 173-176, XP002056653

## Description

The present invention relates to new 1-[4-Arylpiperazin-1-yl]-3-[2-oxopyrrolidin-1-yl] propanes and 1-[4-Arylpiperazin-1-yl]-3-[2-oxopiperidin-1-yl] propanes which can be used as therapeutic agents for hypertension, ischemia, cardiovascular and other adrenergic receptors related disorders, and a process for preparing said novel compounds. More, particularly the present invention relates to 1-[4-Arylpiperazin-1-yl]-3-[2-oxopyrrolidin-1-yl] propanes and 1-[4-Arylpiperazin-1-yl]-3-[2-oxopiperidin-1-yl] propanes, processes for preparing the said compounds and to their use in medicine.

According to one aspect of the invention, there is provided-compounds of formula 1.

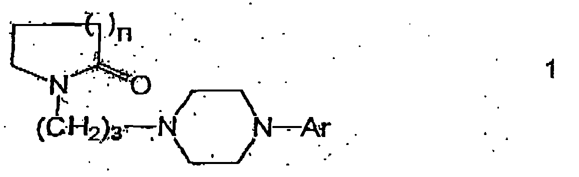

Wherein Ar represents pyridyl, or a phenyl ring substituted by trifluromethyl, halo, alkoxy or alkyl, and n=1 or n=2.

The compounds of the invention have shown to possess antihypertensive activity in different test models. The compounds also prevent post-ischemic reperfusion injury and may be useful in the treatment of hypertension, diseases arising out of adorations/impairment in central/peripheral circulations and adrenergic receptors systems, such as myocardial ischemia, myocardial infarction (MI), angina pectoris, any cardiac surgical interventions renal ischemia, circulatory insufficiency in extremities, stroke and trauma.

A method of preparation of the inventive compounds starts from the condensation of 1-bromo-3-chloropropane with 2-pyrrolidone or 2-piperidone to give the key intermediate 1-chloro-3-[2-oxopyrrolindin-1-yl]propanes (n=1) of formula 3 or 1-chloro-3-[2-oxopiperidin-1-yl]propanes (n=2) followed by its condensation with different 1-substituted piperazines of formula 4 to get the compounds of formula 1 (formulae 2 to 4 are shown in scheme 1 of the accompanying drawings).

Another method which is the subject matter of the co-pending US patent application number ..... is the process of which starts from the condensation of 1-bromo-3-chloropropane with different 1-substituted piperazines of formula 4 to give the 1-chloro-3-(4-substituted piperazin-1-yl)propanes of formula 5 followed by their condensation with 2-pyrrolidone or 2-piperidone of formula 2 to get the compounds of formula 1.

The compounds of the present invention can be used as pharmaceutical compositions comprising compounds of the present invention with a suitable pharmaceutical career. Preferably, these compositions are used to produce antihypertensive and antiischemic activities and contain an effective amount of the compounds useful in the method of the invention. The most preferred compound of the invention is 1-[4-(4-fluorophenyl)piperazin-1-yl]-3-[2-oxopyrrolidin-1-yl]propane.

### BACKGROUND OF THE INVENTION

Hypertension is the most common of all cardiovascular diseases afflicting about 10-20% adult population. Several classes of drugs may be used in the treatment and management of hypertension such as alpha-adrenoceptor antagonists, ACE inhibitors, angiotensin I chymase inhibitors, renin inhibitors, angiotensin II antagonists, vasopressin V₁ antagonists, endothelin antagonists, endothelin-converting enzyme inhibitors, potassium channel activators, calcium channels antagonists, adenosine A₂ agonists, adenosine A₁ antagonists, neutral endopeptidase inhibitiors, dual-action ACE and neutral endopeptidase inhibitors.

These drugs belong to structurally diverse class of heterocyclics including substituted arylpiperazines. In this context, the 1-[4-Arylpiperazin-1-yl]-3-[2-oxopyrrolidin-1-yl]propanes and 1-[4-Arylpiperazin-1-yl]-3-[2-oxopiperidin-1-yl]propanes of the formula 1 are structurally novel compounds and show significant antihypertensive and antiischemic activities. Thus, these compounds would be useful in the treatment of hypertension and in preventing post-ischemic reperfusion injury (ischemia).

The most commonly used antihypertensive drugs are ACE's inhibitors (captopril and related drugs), Ca⁺⁺ channel blocker (nifedipine, verapamil, diltiazen) and peripheral alpha₁-adrenergic antagonist such as prazosin. As these drugs have one or the other side effects, there has been a continuous search for new and safe antihypertensive agents acting by these mechanism and by other novel mechanism which include mainly endothelin antagonists [Gulati, A. and Srimal, R.C. Drug Dev. Res., 26, 361, 1992; Antihypertensive Drugs. The Year's Drug News, 145-167, 1994]. There are no drugs available to prevent post-ischemic reperfusion injury. However, the existing drugs or chemical agents like Ca⁺⁺ channel blockers [Hensch, G. Cardiovascular Res., 26, 14, 1992; Karin Pazyklenk, Robert A. Kloner. Cardiovascular Research, 26, 82, 1992], K_{ATP} openers [Allen W. Gomoll et al., J. Pharmacol. Exp. Ther., 281, 24, 1997; Arthur A.M. Wilde, Cardiovascular Research, 35, 181, 1997] Na⁺/H⁺ exchange inhibitors [Worfgang Scholz et al., Cardiovas. Res., 29, 260, 1995], have been shown to promote myocardial salvage and enhance function recovery *in vivo*, only when given before or during ischemic episode. However, administration of these agents only during reperfusion does not result in cardioprotective activity (Grover, G.J. et al., Cardiovasc. Drugs Ther., 4, 465, 1990 & Eur. J. Pharmacol., 191, 11, 1990; Mizumura, T. et al., Circulation, 92, 1236, 1995). Besides the use of antiischemic agents in prevention of ischemic/reperfusion injury, there is an unmet medical need for agents to treat post-ischemic reperfusion injury which may simulate the real clinical situation of myocardial infarction.

### PRIOR ART

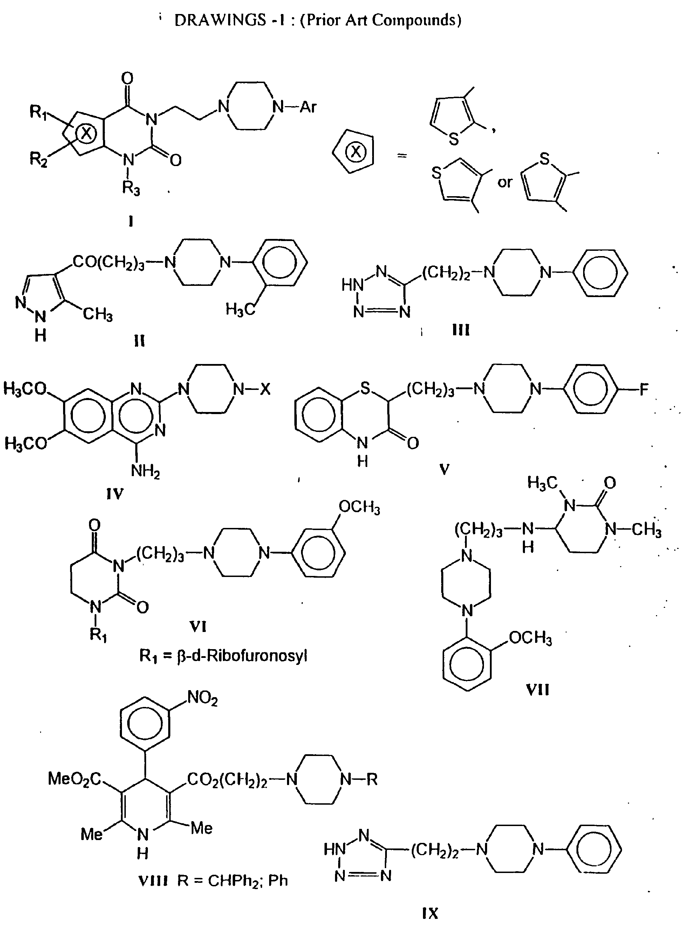

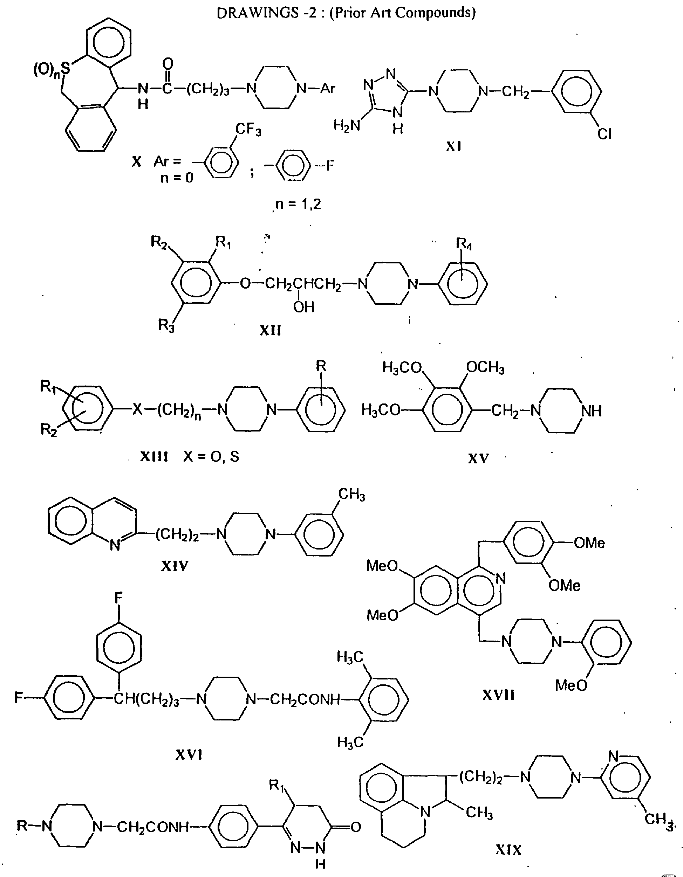

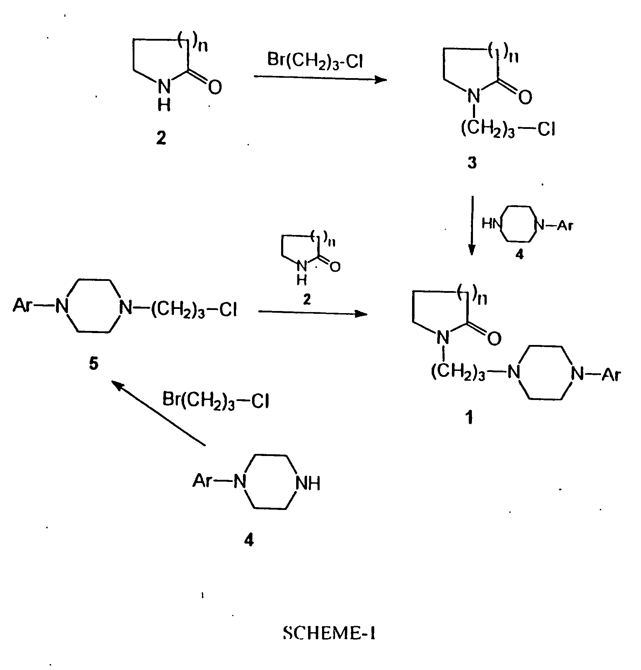

Among a large number of the molecules incorporating arylpiperazines and showing antihypertensive activity, some relevent ones are thienopyrimidine-2,4-diones of formula I of the accompanying drawings-1 (US Patent No. 4,670560, 1989), pyrazoles of formula II of the accompanying drawings - 1 (Arya, V.P. et al., Experentia, 23, 514, 1967), tetrazoles of formula III of the accompanying drawings - 1 (Hayae, S. et al. J. Med. Chem., 10, 400, 1967), prazocin analogs of formula IV of the accompanying drawings - 1 (Luther, R.R. et al., Am. Heart J., 117, 842, 1989; Ames, R.P. & Kiyasu, J.Y. J. Clin. Pharmacol., 29, 123, 1989), 2-[3-[4-(4-fluorophenyl)piperazin-1-yl]propanes]-1,4-benzothiazin-3(4H)-one of formula V of the drawings - 1 (Kajine, M. et al., Chem. Pharm. Bull., 39, 2885, 1991), uracil derivatives of formula VI & VII of the accompanying drawings - 1 (Klmm, Von K. et al., Arzneim. Forsch., 27, 1875, 1977), dihydropyridines of formula VIII of the accompanying drawings - 1 (Suzuki, H. & Saruta, T. Cardiovasc. Drug Rev., 7, 25, 1989; Kubo, K. and Karasawa, A. 10th Int. Cong. Pharmacol., 734, 1987; Drugs of the Future, 14, 291, 1989; Meguro, K. et al., Chem. Pharm. Bull., 33, 3787, 1985; Nakaya, H. et al., Eur. J. Pharmacol., 146, 35, 1988; Kakihand, M. et al., Jpn. J. Pharmacol., 48, 223, 1988; Takenaka, T. et al., Arzneim. Forsch., 26, 2127, 1976; Kajimo, M. et al., Chem. Pharm. Bull., 37, 2225, 1989; Tricerri, S.Z. et al., US Pat. 4,894,460, 1990 : Chem. Abst., 113, 132218b, 1990), zolertine of formula IX of the accompanying drawings - 1 (Arya, V.P. et al., Experientia, 23, 514, 1967; Hayao, S. et al., J. Med. Chem., 10, 400, 1967), thiepin derivatives of formula X of the accompanying drawings - 2 (Uno, H. et al., US Pat. 4,749,703, 1988), triazolylamine of formula XI of the accompanying drawings 1 (Mayer, W.E. et al., J. Med. Chem., 32, 593, 1989), aryloxypropanolamines of formula XII of the accompanying drawings - 2 (Ing, H.R. & Ormerod, W.E., J. Pharm. Pharmacol., 4, 21, 1952; Petrow, V. et al., J. Pharm. Pharmacol., 8, 666, 1956; Moran, N.C. and Perkins, M.E., Pharmacol. Exp. Ther., 124, 223, 1958), aryloxy/thioaryloxy arylpiperazinylpropanes of formula XIII of the accompanying drawings - 2 (Agarwal, S.K. et al., Ind. J. Chem., 21B, 435, 1982; Ind. J. Chem., 21B, 914, 1982; Ind. J. Chem., 30B, 413, 1991; Rao, J. et al., Ind. J. Chem., 26B, 761, 1987; Saxena, A.K. et al., Ind. J. Chem., 32B, 1249, 1993), quinolylethanes of formula XIV of the accompanying drawings - 2 (Murti, A. et al., Ind. J. Chem., 28B, 934, 1989), trimetazidine of formula XV of the accompanying drawings - 2 (Fujita, Y, Jpn. J. Pharmacol., 17, 19, 1976), lidoflazine of formula XVI of the accompanying drawings - 2 (Daenen, W. & Flameng, W., Angiology, 32, 543, 1981), isoquinolylmethyl derivatives of formula XVII of the accompanying drawings - 2 (Nakajiza, T. et al., Arzneim-Forsch., 37, 674, 1987), dihydropyridazinone derivative of formula XVIII of the accompanying drawings - 2 (Yao, F.M. et al., Yaaxue Xuebao, 28, 548, 1993), pyrroloquinoline derivative of formula XIX of the accompanying drawings - 2 (Jasserand, D. et al., Ger. Offen. DE 4,128,015, 1993 : Chem. Abst., 119, 139255f, 1993).

### SUMMARY OF THE INVENTION

The invention relates to 1-[4-Arylpiperazin-1-yl]-3-[2-oxopyrrolidin/piperidin-1- yl]propanes as potential therapeutic agents for hypertension, ischemia, cardiovascular and other adrenergic receptors related disorders ; a process for preparing said compounds and a method of treating hypertension, peripheral viscular diseases, reperfusion injury and ischemic diseases in mammals using said compounds.

Mainly, the present invention centres around the following objects :
(i) The first object of the invention is to provide novel molecules incorporating piperazine flanked on one side by aromatic system and on the other side by 2- (oxopyrrolidin-1-yl)propanes or 2- (oxopiperidin-1-yl)propanes that exhibit better therapeutic efficacy to treat hypertension over the existing antihypertensive agents.
(ii) The second object of the invention is to provide novel 1-(4-arylpiperazin-1-yl)-3- (2-oxopyrrolidin-1-yl)propanes and 1- (4-acrylpiperazin-1-yl) -3- (2-oxopiperidin-1-yl)propanes exhi- biting activity against ischemic reperfusion injury for which there is no agent available till date to the best of the applicants knowledge.
(iii) The third object of the invention is to provide 1-(4- arylpiperazin-1-yl) -3- (2-oxopyrrolidin-1-yl)propanes and 1- (4-arylpiperazin-1-yl)-3- (2-oxopiperidin-1-yl)propanes as therapeutic agents for the diseases arising out of alterations/impairment in central / peripheral circulations and adrenergic receptors systems, such as myocardial ischemia, myocardial infarction (MI), angina pectoris, any cardiac surgical interventions, renal ischemia, circulatory insufficiency in extremities, stroke and trauma.
(iv) The fourth object of the invention is to provide a process for preparing novel 1-(4-aryl-piperazin-1-yl)-3-(2-oxopiperidin-1-yl) propanes and 1-(4-aryl-piperazin-1-yl)-3-(2-oxopiperidin-1-yl) propanes.
(v) The fifth object of the invention relates to a pharmaceutical composition comprising 1-[4-Arylpiperazin-1-yl]-3-[2-oxopyrrolidin / piperidin-1-yl] propanes and pharmaceutically acceptable additive(s) and a process for preparing such composition.
(vi) The sixth object of the invention relates to a method treating hypertension, ischemic reperfusion injury and diseases arising out of alternations / impairment in central / peripheral circulations and adrenergic receptors systems, such as myocardial, ischemia, myocardial infarction (MI), angina pectoris, any cardiac surgical interventions, renal ischemia, circulatory insufficiency in extremities, stroke and trauma.
(vii) The seventh object of the invention is for a method of treating hypertension, ischemia, cardiovascular and other adrenergic receptors related disorders in a patient such as human being and mammals.

To acheive the above and other objects, the present invention provides novel pharmacologically active substances, specifically new 1-(4-arylpiperazin-1-yl)-3-(2-oxopyrrolidin-1-yl)propanes and 1-(4-arylpiperazin-1-yl)-3-(2-oxopiperidin-1-yl)propanes which are used as potential therapeutic agents for hypertension, ischemia, cardiovascular and other adrenergic receptors related disorders.

According to one aspect of the invention, there is provided 1-[4-Arylpiperazin-1-yl] -3- [2-oxopyrrolidinlpiperidin-1-yl] propanes having general formula 1 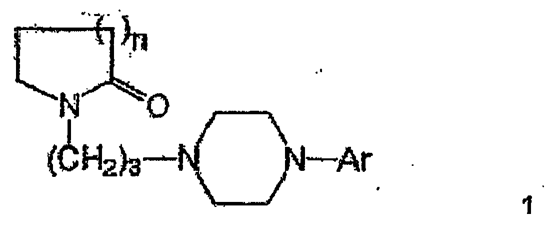 wherein Ar represents pyridyl, or a phenyl ring substituted with, trifluoromethyl, halogen, alkyl, or alkoxy, and n = 1 or n = 2. In an embodiment, the invention comprises the following compounds.
(a) 1-[4-(3-chlorophenyl) piperazin-1-yl]-3-[2-oxopyrrolidin-1-yl] propane.
(b) 1-[4-(4-chlorophenyl)piperazin-1-y1]-3-[2-oxopyrrolidin-1-yl] propane.
(c) 1-[4-(3-fluorophenyl) piperazin-1-yl]-3-[2-oxopyrrolidin-1-yl] propane.
(d) 1-[4-(4-fluorophenyl) piperazin-1-yl]-3-[2-oxopyrrolidin-1-yl] propane.
(e) 1-[4-(4-ethylphenyl)piperazin-1-yl]-3-[2-oxopyrrolidin-1-yl] propane.
(f) 1-[4-(2-ethylphenyl)piperazin-1-yl]-3-[2-oxopyrrolidin-1-yl] propane.
(g) 1-[4-(4-chlorophenyl)piperazin-1-yl]-3-[2-oxopiperidin-1-yl] propane.
(h) 1-[4-(3-chlorophenyl) piperazin-1-yl]-3-[2-oxopiperidin-1-yl] propane.
(i) 1-[4-(4-fluorophenyl)piperazin-1-yl]-3-[2-oxopiperidin-1-yl] propane.
(j) 1-[4-(2-ethylphenyll)piperazin-1-yl]-3-[2-oxopiperidin-1-yl]propane.
(k) 1-[4-(2-methoxyphenyl)piperazin-1-yl]-3-[2-oxopiperidin-1-yl]propane.
(l) 1-[4-(2-pyridyl)piperazin-1-yl]-3-[2-oxopiperidiu-1-yl]propane.
(m) 1-[4-(3-trifluoromethylphenyl)piperazin-1-yl]-3-[2-oxopiperidin-1-yl]propane.

In the specification and claims, the compounds with n = 1 designates 2-oxopyrrolidin-1-yl groups while with n = 2 designates 2-oxopiperidin-1-yl groups. Aryl designates a pyridyl or phenyl, or a phenyl group substituted by one or more alkyl, alkoxy or halogen groups.

A preferred group of compound comprises those in which n = 1 or n = 2, aryl group is 2 or 4-pyridyl, phenyl, or phenyl group substituted by alkyl groups like H, C₂H₅, CF₃, alkoxy like methoxy, halo like chloro, fluoro etc. The compounds of this invention have useful biological activities and have in particular strong antihypertensive and antiischemic activities.

The invention also provides a pharmaceutical composition comprising a compound of formula 1 in admixture with a pharmaceutically acceptable conventional carriers and a process for the preparation of a pharmaceutical composition which comprises bringing a compound of the formula 1 into association with a pharmaceutically acceptable conventional carrier.

According to a second aspect of the invention, there is provided a process for the synthesis of compounds of formula 1 according to the first aspect of the invention, comprising condensing a pyridyl piperazine or an appropriately substituted phenylpiperazine of formula 4, where Ar represents pyridyl, or a phenyl ring substituted by trifluoromethyl, halogen, alkoxy or alkyl, with 1-chloro-3-[2-oxopyrrolidin-1-yl] propane of formula 3 (n=1) or 1-chloro-3-[2-oxopiperidin-1-yl] propane of formula 3 (n=2) in the presence of a base, a catalyst and an organic solvent at a temperature ranging from 70-120°C for a period varying between 8-14 hrs to produce the corresponding 1-[4-substituted arylpiperazin-1-yl]-3-[2-oxopyrrolidin-1-yl] propanes of formula 1 (n=1) or 1-[4-substituted arylpiperazin-1-yl]-3-[2-oxopiperidin-1-yl] propanes of formula 1 (n=2) respectively.

According to a third aspect of the invention, there is provided a pharmaceutical composition comprising a compound of formula 1 according to the first aspect of the invention, in admixture with a pharmaceutically acceptable carrier.

According to a fourth aspect of the invention, there is provided the use of a compound according to the first aspect of the invention, in the manufacture of a medicament for treating hypertension in mammals.

According to a fifth aspect of the invention, there is provided the use of a compound according to the first aspect of the invention, in the manufacture of a medicament for treating peripheral vascular diseases in mammals.

According to a sixth aspect of the invention, there is provided the use of a compound according to the first aspect of the invention, in the manufacture of a medicament for antagonising peripheral alpha-adrenergic receptor in mammals.

According to a seventh aspect of the invention, there is provided the use of a compound according to the first aspect of the invention, in the manufacture of a medicament for treating diseases arising out of alterations/impairment in central/peripheral circulations and adrenergic receptors systems, such as myocardial ischemia, myocardial infarction (MI), angina pectoris, any cardiac surgical interventions, renal ischemia, circulatory insufficiency in extremities, stroke and trauma.

According to a eighth aspect of the invention, there is provided the use of a compound according to the first aspect of the invention, in the manufacture of a medicament for treating reperfusion injury in mammals.

According to a ninth aspect of the invention, there is provided the use of a compound according to the first aspect of the invention, in the manufacture of a medicament for treating ischemic diseases like myocardial infarction (MI), angina pectoris, any cardiac surgical interventions in mammals.

The reaction sequence leading to 1-[4-arylpiperazin-1-yl]-3-[2-oxopyrrolidin-1-yl] propanes or 1-[4-arylpiperazin-1-yl]-3-[2-oxopiperidin-1-yl] propanes is shown in scheme 1 of the accompanying drawings.

It will be noted that according to the foregoing scheme, there are two methods leading to the synthesis of compounds of formula 1 shown earlier.

In the first method the 2-pyrrolidone (**2**, n = 1) or 2-piperidone (**2**, n = 2) is condensed with 1-bromo-3-chloropropane in presence of bases selected from potassium *tert*. butoxide, pulvarised alkali metals selected from sodium or potassium in nonpolar solvents selected from benzene, xylene, toluene at a temperature ranging from 110 to 150°C for 1.15 to 14 hrs to give 1-chloro-3-[2-oxopyrrolidin-1-yl] propane (3, n = 1) or 1-chloro-3-[2-oxo-piperidin-1-yl] propane (3, n = 2) which on condensation with appropriately substituted piperazines, gave the required compounds of formula **1**. This reaction may be carried out in solvents selected from acetone, methylethyl ketone, tetrahydrofuran or dimethylformamide using bases selected from triethylamine, pyridine, sodium or potassium carbonate and catalysts selected from sodium/potassium iodide to improve the yield of the compound of formula 1.

In the second method which is the subject matter of co-pending US patent application number ....., the substituted piperazine (formula 4 of scheme 1 of the accompanying drawings) was condensed with 1-bromo-3-chloropropane in presence of bases selected from sodium or potassium carbonate and catalytic amounts of sodium or potassium iodide in solvents selected from DMF, toluene, xylene etc. at a temperature ranging from 70 to 150°C for 8 to 14 hrs to give 1-chlro-3-(4-substituted piperazin-1-yl)propane (formula 5 of scheme 1 of the accompanying drawings) which on condensation with 2-oxo-pyrrolidine or 2-oxopiperidine in presence of bases selected from potassium *tert*. butoxide or pulverised sodium or potassium in nonpolar solvents selected from xylene, toluene at a temperature ranging from 110 to 150°C for 1.15 to 14 hrs yield the required compounds of formula 1.

The 1-[4-arylpiperazin-1-yl]-3-[2-oxopyrrolidin-1-yl]propanes (1 n=1) and 1-[4-arylpiperazin-1-yl]-3-[2-oxopiperidin-1-yl]propanes (**1** n=2) in free form can, if desired, be converted in to their non-toxic pharmaceutically acceptable acid addition and quaternary ammonium salts. Salts which may be formed comprise, for example, salts with inorganic acids such as hydrochloride, hydrobromide, hydroiodide, sulphate and phosphate. They may also comprise salts with organic acids including mono basic acids such as acetate or propionate and especially those with hydroxy organic acids and dibasic acids such as the citrate, tartarate, malate and maleate. Among useful quaternary ammonium salts are those formed by such alkyl halides as methyl iodine and n-hexyl bromide.

The compounds of the invention show marked antihypertensive alpha-adrenergic blocking and antiischemic activities and can be used as therapeutic agents in diseases arising out of alterations/impairment in central / peripheral circulations and adrenergic receptors systems, such as myocardial ischemia, myocardial infarction (MI), angina pectoris, any cardiac surgical interventions, renal ischemia, circulatory insufficiency in extremities, stroke and trauma as shown for instance by the following data of the compound 1-[4-(4-fluorophenyl)piperazin-1-yl]-3-[2-oxopyrrolidin-1-yl]propane.

### Pharmacological activity

### 1. Acute toxicity (LD₅₀)

- Mice: 147.0 mg/kg i.p. (C.L. = 85.3-253)
562.0 mg/kg p.o. (C.L. = 383-825)

### 2. Effect on blood pressure, heart rate and adrenaline vasopressor response of anaesthetized (pentobarbitone sodium 40 & 60 mg/kg i.p. in cat and rat respectively) normotensive & hypertensive rat & cat model preparations.

| Dose (uMol/kg i.d.) | B.P. fall (%) | Dur. (min.) | Heart rate Pre/post treatment | Adrenaline vasopressure response % inhibition | No. of exp. |
|---|---|---|---|---|---|
| **(A) CAT** | | | | | |
| (i) Naturally occuring hypertensive cat | | | | | |
| 10 | 16 | 110 | 195/195 | 49 | n=4 |
| 20 | 22 | >156.75 | 177/185 | R 26 | n=4 |

| (ii) Normotensive cat | | | | | |
|---|---|---|---|---|---|
| 10 | 22 | 85 | 200/170 | 26 | n=4 |
| 20 | 21 | >102.5 | 185/165 | 34.75 | n=4 |

| **(B) RAT** | | | | | |
|---|---|---|---|---|---|
| (i) Hypertensive rat model preparation | | | | | |
| 5 | 27 | 51 | 346/320 | 33 | n=3 |
| 10 | 22 | 76 | 356/340 | 23 | n=5 |
| 20 | 29 | 100 | 310/275 | 54 | n=1 |

| (ii) Normotensive rat model preparation | | | | | |
|---|---|---|---|---|---|
| 2 i.v. | 25 | 11 | 350/370 | +7 R 22 | n=2 |
| 10 i.v. | 10 | 3 | 315/360 | +14 R 28 | n=2 |
| 20 i.v. | 21 | 27.5 | 315/285 | -9.5 R 42 | n=2 |

| | | | | | |
|---|---|---|---|---|---|
| i.d. = Intraduodenal route; R=Reversal | | | | | |

### 3. Possible site and mechanism of action

### (I) SITE

| | Dose (umol/kg i.d.) | B.P. fall (%) | Dur. (min.) |
|---|---|---|---|
| (i) Spinal transected cat | 2-10 | 14-18 | 15-30 |
| (ii) ICV | 0.34-1.36 | 8-10 | 10-15 |
| (iii) Rat hind limb perfusion | | | |

| | Total dose (ug) | Percent change in flow | |
|---|---|---|---|
| | 10 | no effect | |
| | 25 | +35 (Vasodilatation) | |
| | 50 | +50 (Vasodilatation) | |

### (II) MECHANISM OF ACTION

### (A) In vitro

(i) Isolated aortic strip:
   * Endothelin induced contraction was inhibited significantly.
   * Even after washing the preparation endothelin caused relaxation rather than contraction.
(ii) Isolated Guinea pig ileum preparation endothelin relaxation rather than contraction.
   * Compound showed significant antihistaminic activity (0.5-5.0 ug/ml).
(iii) Langendorffs perfused rat heart preparation:
   * Lower dose of this compound (1 ug) showed some negative chronotropic effect (30% for 10 min.) but higher doses (3-5 ug) showed less negative chronotropic effect (26 & 5% for 14 & 5 min. respectively).
(iv) Konzett and Rossler preparation:
   * Compound showed some antihistaminic activity against histamine induced bronchoconstriction.
(v) Rat aortic ring preparation:
   * NE induced contraction was inhibited by the compound.

### (B) In vivo

Drug antagonism studies at 2 uMol dose i.v. in cat:
(i) Pretreatment with alpha₁-adrenergic receptor blocker, prazosin significantly (90%) reduced antihypertensive effect.
(ii) Pretreatment with Ca⁺⁺ channel blocker, verapamil significantly reduced antihypertensive effect (50%).
(iii) Pretreatment with captopril (an ACE inhibitor) or Dup-753 (an angiotension II-receptor antagonist) also reduced the antihypertensive effect (33%).
(iv) ATP sensitive potassium channel (K_{ATP}) blocker glibenclamide pretreatment only partially reduced the fall in blood pressure.
(v) Pretreatment with, atropine sulphate, mepyramine maleate, propranolol, or yohimbine failed to alter the antihypertensive effect of this compound.

### (4) Comparative antihypertensive effect with clinically used antihypertensive drugs at 2 uMol/kg i.v. dose in cat

| Drug | B.P. fall (%) | Dur. (Min.) |
|---|---|---|
| (i) Verapamil | 55 | 22 |
| (ii) Captopril | 14 | 28 |
| (iii) Compound 1 of formula 1 where Ar=C₆.H₄-4-F, n=1 | 22 | 25 |

### (5) Cardioprotective activity

The most interesting observation is its cardioprotective effect against **myocardial stunning** at a much smaller dose than antihypertensive dose. Further, in Langendorff perfused rat heart preparation subjected to even upto 90 min. global ischemia, the compound at 0.001 ug/ml conc. given at the time of reperfusion revived normal rhythmic contraction started within 2 min. (Table 1) and incidence of reperfusion induced arrhythmia were abolished.

**Table 1: Compound administered at the time of reperfusion at the dose of 0.001 ug/ml on prolong period (90 min.) of ischemic insult.**

| S.No. | Compound | Onset (min.) | Percentage recovery | |
|---|---|---|---|---|
| | | | 15 min. | 30 min. |
| 1. | Control^{a} | 3 | 0.5 | 17.5 |
| 2. | Compound 1 of | 4 | 81.25 | 87.15 |
| | formula 1, where | | | |
| | Ar=C₆H₄-4-F, n=1 | | | |
| 3. | Nifedipine^{a} (10⁻⁶M) | 2.5 | 0.66 | 0.0 |

| | | | | |
|---|---|---|---|---|
| a - Ischemic insult (45 min.) | | | | |

Comparable results for hypotensive/antihypertensive and antiischemic activities were obtained with a number of other compounds of formula 1 (Table 2 & 3).

**Table 2: Effect on blood pressure, heart rate and adrenaline vasopressure response at anaesthetized (pentobarbitone sodium 35 mg/kg(iv)**

| cat | | | | | |
|---|---|---|---|---|---|
| Compound of formula 1 | | Dose (umol/kg i.v.) | B.P. fall % | Dur. (min.) | Adrenaline^{a} vasopressure response % inhibition |
| Ar | n | | | | |
| C₆H₄-4-F | 1 | 2.0 | 25 | 71 | 9 |
| | | 10.0 | 31 | >112 | R |
| C₆H₄-2-C₂H₅ | 1 | 2.0 | 13 | 10 | Pt |
| | | 10.0 | 62 | 10 | - |
| C₆H₄-3-Cl | 1 | 2.0 | 25 | 20 | Pt |
| | | 10.0 | 55 | >57 | - |
| C₆H₄-4-C₂H₅ | 1 | 2.0 | 9 | Tr | - |
| | | 10.0 | 20 | 14 | |
| C₆H₄-3-F | 1 | 2.0 | 18 | 3 | 80 |
| | | 10.0 | 32 | 30 | R |
| C₆H₄-2-OCH₃ | 1 | 2.0 | 19 | 19 | 47 |
| | | 10.0 | 23 | 67 | R |
| 2-Pyridyl | 1 | 2.0 | 22 | 16 | 40 |
| | | 10.0 | 40 | 43 | Pt |
| 2-Pyridyl | 2 | 2.0 | 34 | 53 | 28 |
| | | 10.0 | 42 | >71 | Pt |
| C₆H₄-4-Cl | 2 | 2.0 | 24 | 20 | 20 |
| | | 10.0 | 46 | 25 | 25 |
| C₆H₄-3-Cl | 2 | 2.0 | 27 | 9 | 33 |
| | | 10.0 | 44 | 62 | 10 |
| C₆H₄-3-CF₃ | 2 | 2.0 | - | - | Pt |
| | | 10.0 | 10 | Tr | 25 |
| C₆H₄-2-C₂H₅ | 2 | 2.0 | 35 | 55 | 68 |
| | | 10.0 | 20 | 63 | R |
| C₆H₄-4-F | 2 | 2.0 | 23 | 40 | 46 |
| | | 10.0 | 33 | >84 | R |

| | | | | | |
|---|---|---|---|---|---|
| ^{a} R=Reversal; Pt=potentiation (was within 20%) | | | | | |

**Table 3: Compound administered at the time of reperfusion at a dose of 0.001 ug/ml on brief period (16 min.) of ischemic insult.**

| Compound of formula 1 | | Onset | Percentage recovery | |
|---|---|---|---|---|
| Ar | n | (min.) | 15 min. | 30 min. |
| Control | - | 2.0 | 104.50 | 118.10^{a} |
| C₆H₄-4-F | 1 | 1.5 | 81.25 | 78.12 |
| C₆H₄-2-C₂H₅ | 1 | 3.0 | 106.06 | 136.36 |
| C₆H₄-3-Cl | 1 | 1.0 | 50.00 | 88.80 |
| C₆H₄-4-C₂H₅ | 1 | 2.0 | 103.03 | 60.60 |
| C₆H₄-3-F | 1 | 2.0 | 120.00 | 180.00 |
| C₆H₄-3-Cl | 2 | 3.0 | 31.50 | 39.40 |
| C₆H₄-2-OCH₃ | 1 | 2.0 | 45.40 | 59.09 |
| C₆H₄-4-Cl | 1 | 1.0 | 87.50 | 120.80 |
| 2-Pyridyl | 2 | 2.0 | 47.60 | 71.40 |

| | | | | |
|---|---|---|---|---|
| a - Arrhythmia present | | | | |

The following examples are provided by the way of illustration of the present invention and should in noway be construed as a limitation thereof including the linker (propyl) between pyrrolidone/piperidone and N-arylpiperazine which may be ethyl or butyl.

### Example 1

### (a) Preparation of 1-chloro-3-[2-oxopyrrolidin-1-yl]propane

(i) A mixture of 2-pyrrolidone (1 g, 12.0 mmol) and finely pulverized sodium metal (0.28 g, 12.0 mmol) in dry xylene (60 ml) was heated at 110°C with vigrous stirring, 1-bromo-3-chloropropane (1.8 g, 12.0 mmol) was added to the stirred reaction mixture after 3 hours and the heating at 110°C was continued for 6 hours. The reaction mixture was filtered and xylene was removed under reduced pressure. The residue was distilled under reduced pressure to give **3** (n=1), B.P. 145°C/1 mm., yield 1.52 g (80%). IR (Neat): 2980, 2880, 1710, 1460, 1420, 1280, 1050. ¹H NMR (CDCl₃):1.92-2.18 (m, 4H, 4' & 2-CH₂), 2.40 (t, 2H, J=6.0 Hz, 3'-CH₂), 3.42 (t, 4H, J=6.0 Hz, 5' & 3-CH₂), 3.58 (t, 2H, J=6.0 Hz, 1-CH₂). MS: m/z 161 (M⁺).

| | | | | |
|---|---|---|---|---|
| Mol. formula C₇H₁₂NOCl : | Found: | C, 51.96; | H, 7.48; | N, 8.61 |
| | Calcd. : | C, 52.11; | H, 7.45; | N, 8.69%. |

(ii) A mixture of 2-pyrrolidone (10 g, 120.0 mmol) and finely pulverized sodium metal (2.76 g, 120.0 mmol) in dry xylene (600 ml) was heated at 150°C with vigorous stirring. 1-Bromo-3-chloropropane (18.84 g, 120.0 mmol) was added to the stirred reaction mixture after 30 minutes and the heating at 150°C was continued for 1 hour. The reaction mixture was filtered and xylene was removed under reduced pressure. The residue was distilled under reduced pressure to give **3** (n=1), B.P. 145°C/1 mm., yield 16.09 g (85%).
(iii) A mixture of 2-pyrrolidone (2 g, 23.0 mmol) and finely pulverized sodium metal (0.529 g, 23.0 mmol) in dry toluene (120 ml) was heated at 120°C with vigorous stirring. 1-Bromo-3-chlropropane (3.97 g, 25.0 mmol) was added to the stirred reaction mixture after 6-7 hours and the heating at 120°C was continued for 7 hours. The reaction mixture was filtered and toluene was removed under reduced pressure. The residue was distilled under reduced pressure to give the compound **3** (n=1), B.P. 145°C/1 mm., yield 2.86 g (75%).
(iv) A mixture of 2-pyrrolidone (1 g, 12.0 mmol) and finely pulverized potassium metal (0.47 g, 12.0 mmol) in dry xylene (60 ml) was heated at 150°C with vigorous stirring. 1-Bromo-3-chloropropane (1.8 g, 12.0 mmol) was added to the stirred reaction mixture after 20 minutes and the heating at 150°C was continued for 1 hour. The reaction mixture was filtered and xylene was removed under reduced pressure. The residue was distilled under reduced pressure to give 3 (n=1), B.P.145 ° C/1 mm., yield 1.43 g (75%).
(v) A mixture of 2-pyrrolidone (1 g, 12.0 mmol) and potassium tert. butoxide (1.34 g, 12.0 mmol) in dry xylene (60 ml) was heated at 150°C with vigorous stirring, 1-Bromo-3-chloropropane (1.8 g, 12.0 mmol) was added to the stirred reaction mixture after 2 hours and the heating at 150°C was continued for 3 hours. The reaction mixture was filtered and xylene was removed under reduced pressure. The residue was distilled under reduced pressure to give **3** (n=1), B.P. 145 °C/1 mm., yield 1.24 g (65%).

### (b) Preparation of 1-chloro-3-(2-oxopiperidin-1-yl)propane

A mixture of 2 piperidone (1.19 g, 12.0 mmol) and finely pulverized sodium metal (0.28 g, 12.0 mmol) in dry xylene (70 ml) was heated at 110°C with vigorous stirring. 1-bromo-3-cmoropropane (1.89 g, 12.0 mmol) was added to the stirred reaction mixture after 3 hours and the heating at 110 ° C was continued for 6 hours. The reaction mixture was filtered and xylene was removed under reduced pressure. The residue was chromatographed on silica gel using hexane and chloroform as eluant to get **3** (n-2), B.P. 91 °C/0.01 mm., yield 1.33 g (63.33%). Ir (Neat) : 3862, 3298, 2950, 2474, 2324, 1640, 1478, 1432, 1336, 1884, 1096, 754. ¹H NMR (CDCl₃) : 1.79-2.36 (m, 8H), 3.15 - 3.67 (m, 6H). MS: m/z 175 (M⁺).

| | | | | |
|---|---|---|---|---|
| Mol. formula C₈H₁₄NOCl: | Found : | C, 54.90; | H, 8.28; | N, 8.25 |
| | Calcd. : | C, 54,69; | H, 8.03; | N, 7.97%. |

### Example 2

(a) Preparation of 1-[4-(3-chlorophenyl)pipuain-1-yl]-3-[2-oxopyrrolidin-1-yl] propane of the formula 1, where Ar=C₆H₄-3-Cl, n=1
A mixture of 1-chloro-3-[2-oxopyrrolidin-1-yl]propane (1 g, 6.2 mmol), 1-(3-chlorophenyl)piperazine (1.22 g, 6.2 mmol) anhydrous Na₂CO₃ (0.33 g, 3.1 mmol) and NaI (0.093 g, 0.6 mmol) in dry DMF (5 ml) was stirred at 70°C for 14 hrs. The reaction mixture was cooled, poured on water (20 ml) and the separated residue was extracted with CHCl₃ (2 x 25 ml). The extracts were dried over Na₂SO₄ and concentrated under reduced pressure to give 1-[4-(3-chloropherryl)piperazin-1-yl]-3-[2-oxopyrrolidin-1-yl]propane as an oil which was purified by flash column chromatography over silica gel using chloroform as eluent, yield 1.50 g (75%). IR (Neat): 3020, 2820, 1660, 1590, 1450, 1210, 730. ¹H NMR (CDCl₃): 1.30-2.60(m, 12H, 3', 4', 2, 1 & 2xN-CH₂), 2.40-3.50(m, 8H, 5', 3 & 2xN-CH₂), 6.40-7.20(m, 4H, ArH). MS; m/z 321 (M⁺) 323 (M+2).

| | | | | |
|---|---|---|---|---|
| Mol, Formula C₁₇H₂₄ClN₃O : | Found : | C, 63.58; | H, 7.82; | N, 13.17 |
| | Calcd. : | C, 63.44; | H, 7.52; | N, 13.06%. |

(b) A mixture of 2-pyrrolidone (1 g, 12 mmol) and finely pulverized sodium metal (0.28 g, 12.0 mmol) in dry toluene (60 ml) was heated at 120°C with vigorous stirring for 6 hours, 1-4[4-(3-chlorophenyl)piperazin-1-yl]-3-chloropropane (3.26 g, 12.0 mmol) was added to this reaction mixture and the reaction mixture was heated under stirring at 120°C for 7 hour. The reaction mixture was filtered and toluene was removed under reduced pressure. The residue was chromatographed over flash silica gel using chloroform as eluent to give the title product, yield 2.65 g (70%), oil.
(c) A mixture of 1-chloro-3-[2-oxopyrrolidin-1-yl)propane (1.0 g, 6.2 mmol), 1-(3-chlorophenyl)piperazine (1.22 g, 6.2 mmol), anhydrous Na₂CO₃ (0.33 g, 3.1 mmol) and NaI (0.093 g, 0.6 mmol) in dry toluene (10 ml) was stirred at 110°C for 12 hrs. The solvent was removed at reduced pressure and residue was poured on water (20 ml). The separated residue was extracted with ethylacetate (3x20 ml), dried over Na₂SO₄ and concentrated under reduced pressure to give 1-[4-(3-chlorophenyl)piperazin-1-yl]-3-[2-oxopyrrolidin-1-yl]propane as an oil which was purified by flash column chromatography over silica gel using chloroform as eluent, yield 0.60 g (30.0%).
(d) A mixture of 1-chloro-3-[2-oxopyrrolidin-1-yl]propane (1 g, 6.2 mmol), 1-(3-chlorophenyl)piperazirie (1.22 g, 6.2 mmol) anhydrous Na₂CO₃ (0.33 g, 3.1 mmol) and NaI (0.093 g, 0.6 mmol) in dry xylene (15 ml) was stirred at 150°C for 14 hrs. The solvent was removed at reduced pressure and residue was poured on water (30 ml). The separated residue was extracted with dichloromethane (2x25 ml), dried over Na₂SO₄ and concentrated to give 1-[4-(3-chlorophenyl)piperazin-1-yl]-3-[2-oxopyrrolidin-1-yl]propane as an oil which was purified by flash column chromatography over silica gel using chloroform as eluent; yield 0.72 g (36%).

### Example 3

(a) Preparation of 1-[4-(chlorophenyl)piperazin-1-yl]-3-[2-oxopyrrolidin-1-yl] propane of the formula 1, where Ar=C₆H₄-4-Cl, n=1
A mixture of 1-chloro-3-[2-oxopyrrolidin-1-yl]propane (1 g, 6.2 mmol), 1-4(chlorophenyl)piperazine (1.22 g, 6.2 mmol) anhydrous Na₂CO₃ (0.33 g, 3.1 mmol) and NaI (0.09 g, 0.6 mmol) in dry DMF (5 ml) was stirred at 70°C for 12 hrs. The reaction mixture was cooled, poured on water (20 ml) and the separated residue was extracted with CHCl₃ (2x25 ml). The extracts were dried over Na₂SO₄ and concentrated under reduced pressure to give 1-[4-(4-chlorophenyl)piperazin-1-yl]-3-[2-oxopyrrolidin-1-yl]propane which was purified by flash column chromatography over silica gel using chloroform as eluent, yield 1.48 g (74%), M.P. 70-80°C. IR (KBr): 3440, 2820, 1660, 1490, 1230, 1130, 810. ¹H NMR (CDCl₃): 1.50-2.80 (m, 12H, 3', 4', 2, 1 & 2xM-CH₂), 3.00-3.60 (m, 8H, 5', 3 & 2xN-CH₂), 6.80 (d, 2H, J=9.0 Hz, ArH, o to Cl), 7.20 (d, 2H, J=9.0 Hz, ArH, m to Cl). MS: m/z 321 (M⁺) 323 (M+2).

| | | | | |
|---|---|---|---|---|
| Mol. formula C₁₇H₂₄ClN₃O : | Found: | C, 63.84; | H, 7.32; | N, 13.12 |
| | Calcd.: | C, 63,44; | H, 7.52; | N, 13.06%. |

(b) A mixture of 2-pyrrolidone (1 g, 12 mmol) and finely pulverized sodium metal (0.28 g, 12.0 mmol) in dry xylene (60 ml) was heated at 140°C with vigorous stirring, 1-[4-chlorophenyl)piperazin-1-yl]-3-chloropropane (3.26 g, 12.0 mmol) was added to the stirred reaction mixture after 30 minutes and the heating at 140°C was continued for 1 hour. The reaction mixture was filtered and xylene was removed under reduced pressure to give **3** which was purified by flash column chromatography over silica gel using chloroform as eluent, yield 2.83 g (75%), M.P. 78-80°C.
(c) A mixture of 2-pyrrolidone (1 g, 12 mmol) and finely pulverized potassium metal (0.47 g, 12.0 mmol) in dry xylene (60 ml) was heated at 150°C with vigorous stirring, 1-[4-(4-cblorophenyl)piperazin-1-yl]-3-chloropropane (3.26 g, 12.0 mmol) was added to the stirred reaction mixture after 20 minutes and the heating at 150°C was continued for 1 hour. The reaction mixture was filtered and xylene was removed under reduced pressure to give **3** which was purified by flash column chromatography over silica gel using chloroform as eluent, yield 2.78 g (73.5%), M.P. 78-80°C.
(d) A mixture of 2-pyrrolidone (1 g, 12 mmol) and finely pulverized potassium tert. butoxide (1.34 g, 12.0 mmol) in dry xylene (60 ml) was heated at 150°C with vigorous stirring, 1-[4-(4-chlorophenyl)piperazin-1-yl]-3-chloropropane (3.26 g, 12.0 mmol) was added to the stirred reaction mixture after 2 hours and the heating at 150°C was continued for 4 hours. The reaction mixture was filtered and xylene was removed under reduced pressure to give **3** which was purified by flash column chromatography over silica gel using chloroform as eluent, yield 2.46 g (65%), M.P. 78-80°C.

### Example 4

a) Preparation of 1-[4-(3-fluorophenyl)piperazin-1-yl]-3-[2-oxopyrrolidin-1-yl] propane of the formula 1, where Ar=C₆H₄-3-F, n=1
A mixture of 1-chloro-3-[2-oxopyrrolidin-1-yl]propane (1g. 6.2 mmol), 1-(3-fluorophenyl)piperazine (1.12 g, 6.2 mmol), anhydrous K₂CO₃ (0.434 g, 3.1 mmol) and KI (0.10 g, 0.6 mmol) in dry DMF (5 ml) was stirred at 90°C for 12 hrs. The reaction mixture was cooled, poured on water (25 ml) and the separated residue was extracted with CHCl₃ (2x25 ml). The extracts were dried over Na₂SO₄ and concentrated under reduced pressure to give 1-[4-(3-fluorophenyl)piperazin-1-yl]-3-[2-oxopyrrolidin-1-yl]propane as an oil which was purified by flash column chromatography over silica gel using chloroform as eluent, yield 1.32 g (69.6%). IR (Neat): 2920, 2800, 1650, 1440, 1240, 1150, 740. ¹H NMR (CDCl₃): 1.50-2.70 (m, 12H, 3', 4', 2,1 & 2xN CH₂), 3.00-3.60 (m, 8H, 5', 3 & 2xN-CH₂), 6.30-6.80 (m, 4H, ArH). MS: m/z 305 (M⁺) 307 (M+2).

| | | | | |
|---|---|---|---|---|
| Mol. formula C₁₇H₂₄FN₃O: | Found: | C, 67.22; | H, 7,72; | N, 13.97 |
| | Calcd.: | C, 66.88; | H, 7.92; | N, 13.76%. |

(b) A mixture of 2-pyrrolidone (1 g, 12 mmol) and finely pulverized sodium metal (0.28 g, 12.0 mmol) in dry xylene (60 ml) was heated at 150°C with vigorous stirring. 1-[4-(3-fluorophenyl)piperazin-1-yl]-3-chloropropane (3.07 g, 12.0 mmol) was added to the stirred reaction mixture and the heating at 150°C was continued for 1 hour. The product was obtained by the similar method as in Example 3, yield 2.94 g (82%), oil.

### Example 5

a) Preparation of 1-[4-fluorophenyl)piperazin-1-yl]-3-[2-oxopyrrolidin-1-yl] propane of the formula 1, where Ar=C₆H₄-4-F, n=1
A mixture of 1-chloro-3-[2-oxopyrrolidin-1-yl]propane (4 g, 25.0 mmol), 1-(4-fluorophenyl)piperazine (4.47 g, 25.0 mmol), anhydrous Na₂CO₃ (1.325 g, 12.5 mmol) and NaI (0.38 g, 2.5 mmol) in dry DMF (20 ml) was stirred at 120°C for 8 hrs. The reaction mixture was cooled, poured on water (30 ml) and the separated residue was extracted with CHC₃ (2x30 ml). The extracts were dried over Na₂SO₄ and concentrated under reduced pressure to give 1-[4-(4-fluorophenyl)piperazin-1-yl]-3-[2-oxopyrrolidin-1-yl]-propane as an oil which was purified by flash column chromatography over silica gel using chloroform as eluent, yield 5.72 g (75.5%). IR (Neat): 2920, 2820, 1660, 1500, 1250, 730. ¹H NMR (CDCl₃): 1.50-2.80(m, 12H, 3', 4', 2, 1 & 2xN-CH₂), 3.00-3.60(m, 8H, 5', 3 & 2xN-CH₂), 6.70-7.10 (m, 4H, ArH). MS: m/z 305 (M⁺).

| | | | | |
|---|---|---|---|---|
| Mol. formula C₁₇H₂₄FN₃O: | Found: | C, 66.55; | H, 8.07; | N,13.69 |
| | Calcd.: | C, 66.86; | H, 7.92; | N,13.76%. |

(b) A mixture of 2-pyrrolidone (3 g, 35 mmol) and finely pulverized sodium metal (0.81 g, 35.0 mmol) in dry xylene (180 ml) was heated at 150°C with vigorous stirring, 1-[4-(4-fluorophenyl)piperazin-1-yl]-3-chloropropane (8.96 g, 35.0 mmol) was added to the stirred reaction mixture after 30 minutes and the heating at 150°C was continued for 1 hour. The reaction mixture was filtered and xylene was removed under reduced pressure to give **3** which was purified by flash column chromatography over silica gel using chloroform as eluent, yield 9.15 g (85%), oil.

### Example 6

a) Preparation of 1-[4-(ethylphenyl)piperazin-1-yl]-3-[2-oxopyrrolidin-1-yl] propane of the formula 1, where Ar=C₆H₄-4-C₂H₅, n=1
A mixture of 1-chloro-3-[2-oxopyrrolidin-1-yl]propane (2.0 g, 12.0 mmol), 1-(4-ethylphenyl)piperazine (2.36 g, 12.0 mmol), anhydrous Na₂CO₃ (0.658 g, 6.2 mmol) and NaI (0.18 g, 1.2 mmol) in dry DMF (10 ml) was stirred at 80°C for 12 hrs. The reaction mixture was cooled, poured on water (30 ml) and the separated residue was extracted with CHCl₃ (2x30ml). The extracts were dried over Na₂SO₄ and concentrated under reduced pressure to give 1-[4-(4-ethylphenyl)piperazin-1-yl]-3-[2-oxopyrrolidin-1-yl]propane as an oil which was purified by flash column chromatography over silica gel using chloroform as eluent, yield 2.43 g (62.0%). IR (Neat): 2940, 2800, 1660, 1440, 1000, 900, 800. ¹H NMR (CDCl₃): 1.20 (t, 3H, J=6.0 Hz. CH₂CH₃, 1.60-2.80 (m, 14H, 3', 4', 2, 1 & 2xN-CH₂, CH₂CH₃), 3.00-3.60 (m, 8H, 5', 3 & 2xN-CH₂). MS: m/z 315 (M⁺).

| | | | | |
|---|---|---|---|---|
| Mol. formula C₁₉H₂₉N₃O: | Found: | C, 71.94; | H, 9.16; | N, 18.9% |
| | Calcd.: | C, 72.34; | H, 9.27; | N, 18.39%. |

(b) A mixture of 2-pyrrolidone (2 g, 24 mmol) and finely pulverized sodium metal (0.56 g, 24.0 mmol) in dry xylene (120 ml) was heated at 150°C with vigorous stirring, 1-[4-(4-ethylphenyl)piperazin-1-yl]-3-chloropropane (6.38 g, 24.0 mmol) was added to the stirred reaction mixture after 30 minutes and the heating at 150°C was continued for 1 hour. The product was obtained by the similar method as in Example 3, yield 6.42 g (78%), oil.

### Example 7

a) Preparation of 1-[4-(2-ethylphenyl)piperazin-1-yl]-3-[2-oxopyrrolidin-1-yl] propane of the formula 1, where Ar=C₆H₄-2-C₂H₅, n=1
A mixture of 1-chloro-3-[2-oxopyrrolidin-1-yl]propane (2.0 g, 12.0 mmol), 1-(2-ethylphenyl)piperazine (2.36 g, 12.0 mmol), anhydrous Na₂CO₃ (0.658 g, 6.2 mmol) and NaI (0.18 g, 1.2 mmol) in dry DMF (10 ml) was stirred at 80°C for 12 hrs. The reaction mixture was cooled, poured on water (30 ml) and the separated residue was extracted with CHCl₃ (2x20ml). The extracts were dried over Na₂SO₄ and concentrated under reduced pressure to give 1-[4-(4-ethylphenyl)piperazin-1-yl]-3-[2-oxopyrrolidin-1-yl]propane as an oil which was purified by flash column chromatography over silica gel using chloroform as eluent, yield 2.17 g (60.0%). IR (Neat): 2940, 2820, 1660, 1430, 1010, 900, 800. ¹H NMR (CDCl₃): 1.24(t, 3H, J=6 Hz, CH₂-CH₃), 1.68-1.85 (m, 2H, 4'-CH₂), 1.98-2.10 (m, 2H, 2-CH₂), 2.34-2.50 (m, 4H, 3H & 1-CH₂), 2.62 (bs, 4H, 2xN-CH₂), 2.68 (q, 2H, CH₃-CH₂), 2.94 (t, 4H, J=6.0 Hz, 2xN-CH₂), 2.35 (t, 2H, J=6.0 Hz, 3-CH₂), 3.42 (t, 2H, J=6.0 Hz, 5',CH₂), 7.00-7.28 (m, 4H, Ar-H). MS: m/z 315 (M⁺).

| | | | | |
|---|---|---|---|---|
| Mol formula C₁₉H₂₉N30 : | Found: | C, 72.64; | H, 9.11; | N, 18.25% |
| | Calcd: | C, 72.34; | H, 9.27; | N, 18.39% |

(b) A mixture of 2-pyrrolidone (1 g, 12 mmol) and finely pulverized sodium metal (0.28 g, 12.0 mmol) in dry xylene (60 ml) was heated at 150°C with vigorous stirring, 1-[4-(3-ethylphenyl)piperazin-1-yl]-3-chloropropane (3.19 g, 12.0 mmol) was added to the stirred reaction mixture after 30 minutes and the heating at 150°C was continued for 1 hour. The product was obtained by the similar method as in Example 3, yield 3.13 g (76%), oil.

### Example 8

a) Preparation of 1-[4-(4-cblorophenyl)piperazin-1-yl]-3-[2-oxopipexidin-1-yl] propane of the formula 1, where Ar=C₆H₄-4-Cl, n=2
A mixture of 2-piperidone (1 g, 10 mmol) and finely pulverized sodium metal (0.23 g, 10 mmol) in dry xylene (60 ml) was heated at 140°C with vigorous stirring, 1-[4-(4-chlorophenyl)piperzin-1-yl]-3-chloropropane (2.72 g, 10 mmol) was added to the stirred reaction mixture after 30 minutes and the heating at 140°C was continued for 1 hour. The reaction mixture was filtered and xylene was removed under reduced pressure to give **3** which was purified by flash column chromatography over silica gel using chloroform as eluant, yield 2.54 g (75%), m.p. 106°C. IR (neat): 3761, 3408, 3017, 2957, 2882, 2826, 2785, 2502, 1659, 1599, 1499, 1456, 1385, 1346, 1219, 1148, 1105, 760, 667. PMR (CDCl₃) : 0.89-1.80 (m, 6H, 4',5, & 2-CH₂), 2.33-2.65(m, 8H, 3', 1 & 2xN-CH₂), 3.15-3.62(m, 8H, 6',3 & 2xN-CH₂), 6.81-7.00 (2xdd, 4xArH); MS: m/z 337 (M+2).

| | | | | |
|---|---|---|---|---|
| Mol formula C₁₈H₂₆N₃OCl : | Found: | C, 64.23; | H, 7.87; | N, 12.24 |
| | Calcd.: | C, 64.37; | H, 7.80; | N, 12.51% |

(b) A mixture of 2-piperidone (1.0 g, 10 mmol) and finely pulverized potassium tert. butoxide (1.12 g, 10 mmol) in dry xylene (60 ml) was heated at 150°C with vigorous stirring, 1-[4-(4-chlorophenyl)piperazin-1-yl]-3-chloropropane (2.72 g), 10 mmol) was added to the stirred reaction mixture after 2 hours and the heating at 150°C was continued for 4 hours. The reaction mixture was filtered and xylene was removed under reduced pressure to give **3** which was purified by flash column chromotography over silica gel using chloroform as eluant, yield 2.17 g (64%), m.p. 106°C.

### Example 9

a) Preparation of 1-[4-(3-chlorophenyl)piperazin-1-yl]-3-[2-oxopiperidin-1-yl] propane of the formula 1, where Ar=C₆H₄-3-Cl, n-2
A mixture of 2-piperidone (1.0 g, 10 mmol) and finely pulverized sodium metal (0.23 g, 10 mmol) in dry toluene (60 ml) was heated at 120°C with vigorous stirring for 6 hours, 1-[4-(3-chlorophenyl)piperazin-1-yl]-3-chloropropane (2.72 g, 10 mmol) was added to this reaction mixture and the reaction mixture was heated under stirring at 120°C for 7 hours. The reaction mixture was filtered and toluene was removed under reduced pressure. The residue was chromatographed over flash silica gel using chloroform as eluant to give **3**, yield 2.59 g (75.5%), oil IR (Neat): 3406, 2951, 2878, 1626, 1597, 1491, 1454, 1383, 1352, 1219, 1142, 1103, ¹H NMR (CDCl₃): 1.17-1.84 (m, 6H, 2, 4' & 5'-CH₂), 2.27-2.65 (m, 8H, 1, 3' & 2xN-CH₂) 3.15-3.37 (m, 8H, 6', 3 & 2xN-CH₂), 6.68-.6.79 (dd, 2xArH), 7.05-7.11 (t, 1xArH), 7.19 (s, 1xArH). MS: m/z 33 5 (M⁺).

| | | | | |
|---|---|---|---|---|
| Mol. formula C₁₈H₂₆N₃OCl: | Found: | C, 64.17; | H, 7.92; | N, 12.21 |
| | Calcd.: | C, 64.37; | H, 7.80; | N. 1251% |

### Example 10

a) Preparation of 1-[4-(4-fluorophenyl)piperazin-1-yl]-3-[2-oxopiperidin-1-yl] propane of the formula 1, where Ar=C₆H₄-4-F, n=2
A mixture of 2-piperidone (2 g, 20 mmol) and finely pulverized sodium metal (0.46 g, 20 mmol) in dry xylene (120 ml) was heated at 150°C with vigorous stirring, 1-[4-(4-fluorophenyl)piperazin-1-yl]-3-chloropropane (5.17 g, 20 mmol) was added to the stirred reaction mixture after 30 minutes heating at 150°C was continued for 1 hour. The reaction mixture was filtered and xylene was removed under reduced pressure to give **3** which was purified by flash column chromatography over silica gel using chloroform as elauant, yield 4.64 g (72%), oil. IR (Neat): 3408, 3014, 2931, 2880, 2825, 1626, 1508, 1458, 1219, 824, 760, 667. ¹H NMR (CDCl₃): 1.72-1.86 (m, 6H, 4', 5' & 2-CH₂), 2.37-2.69 (m, 8H, 3', 1 & 2xN-CH₂), 3.12-3.46(m, 8H, 6', 3 & 2xN-CH₂), 6.83-7.00(2xdd, 4xArH). MS: 319 (M⁺).

| | | | | |
|---|---|---|---|---|
| Mol formula C₁₉H₂₆N₃OF: | Found: | C, 71.23; | H, 8.26; | N, 12.95 |
| | Calcd.: | C, 71.47; | H, 8.15; | N, 13.17% |

(b) A mixture of 1-chloro-3-[2-oxopiperidin-1-yl]propane (2.1 g, 12 mmol), 1-4(fluorophenyl)piperazine (2.16 g, 12 mmol), anhydrous Na₂CO₃ (0.618 g, 6.2 mmol) and Nal (0.18 g, 1.2 mmol) in dry DMF (10 ml) was stirred at 80°C for 14 hrs. The reaction mixture was cooled, poured on water (30 ml) and the separated residue was extracted with CHCl₃) (2x35 ml). The extract were dried over NO₂SO₄ and concentrated under reduced pressure to give 1-[4-(4-fluorophenyl)piperazin-1-yl]-3-[2-oxopiperadin-1-yl]propane as an oil which was purified by column chromatography over silica gel using chloroform as eluent, yield 2.40 g (63.0%).

### Example 11

### Preparation of 1-[4-(2-ethylphenyl)pipetazin-1-yl]-3-[2-oxopiperidin-1-yl] propane of the formula 1, where Ar=C₆H₄-2-C₂H₅, n=2

A mixture of 2-piperidone (1.0 g, 10 mmol) and finely pulverized sodium metal (0.23 g, 10 mmol) in dry xylene (60 ml) was heated at 150°C with vigorous stirring, 1-[4-(2-ethylphenyl)piperazin-1yl]-3-chloropropane (2.66 g, 10 mmol) was added to the stirred reaction mixture after 30 minutes and the heating at 150°C was continued for 1 hour. The reaction mixture was filtered and xylene was removed under reduced pressure to give **3** which was purified by flash column chromatography on silica gel using chloroform as eluant, yield 2.49 g (75%), oil. IR (Neat): 3680, 3440, 3000, 2960, 2870, 2820, 1620, 1490, 1450, 1350,1210, 1140, 1005, 920, 725. ¹H NMR (CDCl₃): 1.00 (t, 3H, CH₂CH₃), 1.20-1.90(m, 6H, 4', 5' & 2-CH₂), 2.20-3.50(m, 18H, 3', 6', 3, 1 & 4xN-CH₂, CH₂CH₃), 7.00-7.20(m, 4H, ArH). MS: m/z 329 (M⁺).

| | | | | |
|---|---|---|---|---|
| Mol. formula C₂₀H₃₁N₃O: | Found: | C, 72.67; | H, 9.60; | N, 12.49 |
| | Calcd.: | C, 72.95; | H, 9.42; | N, 12.77%. |

### Example 12

### Preparation of 1-[4-(2-methoxyphenyl)piperazin-1-yl]-3-[2-oxopiperidin-1-yl] propane of the formula 1, where Ar=C₆H₄-2-OCH₃, n=2

A mixture of 2 piperidone (1.0 g, 10 mmol) and finely pulverized potassium metal (0.40 g, 10 mmol) in dry xylene (60 ml) was heated at 150°C with vigorous stirring, 1-[4-(2-methoxyphenyl)piperarin-1-yl]-3-chloropropane (2.52 g, 10 mmol) was added to the stirred reaction mixture after 20 minutes and the heating at 150°C was continued for 1 hour. The reaction mixture was filtered and xylene was removed under reduced pressure to give **3** which was purified by flash column chromatography over silica gel using chloroform as eluant, yield 2.41 g (72%), oil. IR (Neat): 2945, 1626, 1500, 1460, 1242, 908, 735, 648. PMR (CDCl₃): 1.69-1.84 (m, 6H, 4', 5' & 2-CH₂, 2.26-2.70(m, 8H 3', 1 & 2xN-CH₂), 3.07-3.36 (m, 8H, 6', 3 & 2xN (CH₂), 3.75(s, 3H, OCH₃), 6.76-6.95 (m, 4H, ArH). MS: m/z 331 (M⁺).

| | | | | |
|---|---|---|---|---|
| Mol. formula C₁₉H₂₉N₃O₂: | Found: | C, 68.61; | H, 8.92; | N, 12.56 |
| | Calcd.: | C, 68.85; | H, 8.81; | N,12.68%. |

### Example 13

### Preparation of 1-[4-(2-pyridyl)piperazin-1-yl]-3-[2-oxopiperidin-1-yl] propane of the formula 1, where Ar=2-pyridyl, n=2

A mixture of 2-piperidone (2 g, 20 mmol) and finely pulverized sodium metal (0.46 g, 20 mmol) in dry xylene (120 ml) was heated at 150°C with vigorous stirring, 1-[4-(2-pyridyl)piperazine-1-yl]-3-chloropropane (4.78 g, 20 mmol) was added to the reaction mixture after 30 minutes and the heating at 150°C was continued for 1 hour. The reaction mixture was filtered and xylene was removed under reduced presure to give 3 which was purified by flash column chromatography over silica gel using chloroform as eluant, yield 4.39 g (72%), oil. IR (Neat): 3420, 2940, 2800, 1629, 1580, 1470, 1420, 1230, 1150, 1125, 960, 720. ¹H NMR (CDCl₃):1.50-2.20 (m, 6H, 4', 5' & 2-CH₂), 2.25-2.80 (m, 8H, 3', 1 & 2xN-CH₂), 3.10-4.80 (m, 8H, 6', 3 & 2xN-CH₂), 6.48-6.75 (m, 2H, 3,5-pyridyl H), 7.40 (m, 1H, 4-pyridyl H), 8.15 (m, 1H, 6-pyridyl H). MS: m/z 302 (M⁺).

| | | | | |
|---|---|---|---|---|
| Mol. formula C₁₇H₂₆N₄O; | Found: | C, 67.32; | H, 8.49; | N, 18.38 |
| | Calcd.: | C, 67.52; | H, 8,67; | N, 18.53%. |

### Example 14

### Preparation of 1-[4-(3-trifluoromethylphenyl)pipcrazin-1-yl]-3-[2-oxopiperidin-1-yl]propane of the formula 1, where AR=C₆H₄-3-CF₃, n=2.

A mixture of 2-piperidone (1.0 g, 10 mmol) and finely pulverized sodium metal (0.23 g, 10 mmol) in dry xylene (60 ml) was heated at 150 °C with vigorous stirring, 1-[4-(3-CF₃-phenyl)piperazin-1-yl]-3-chloropropane (3.06 g, 10 mmol) was added to the stirred reaction mixture after 30 minutes and the heating at 150°C was continued for 1 hour. The reaction mixture was filtered and xylene was furnished by flush column chromatography on silica gel using CHCl₃ as eluant, yield 2.80 g (75%), oil. IR (Neat): 3402, 3015, 2951, 2880, 2827, 2785, 1622, 1449, 1450, 1352, 1315, 1217, 1167, 1126, 1076, 997, 951. ¹H NMR (CDCl₃) : 1.75-1.93 (m, 6H, 4', 5' & 2-CH₂), 2.35-2.75 (m, 8H, 8',1 & 2xN-CH₂), 3.310-3.84 (m, 8H, 6', 3 & 2xN-CH₂), 7.04-7.10 (dd, 2H, 4, 6-ArH), 7.26 (s, H, 2-ArH), 7.32-7.37 (t, H, 5-ArH). MS: m/z 369 (M⁺).

| | | | | |
|---|---|---|---|---|
| Mol. formula C₁₉H₂₆N₃OF₃: | Found: | C, 61.48; | H, 7.23; | N, 11.21 |
| | Calcd.: | C, 61.79; | H, 7.05; | N, 11.38%. |

### Example 15

### Antihypertensive/hypotensive activity

(a) Cats (2.6-4.0 kg) of either sex anaesthetized with pentobarbitone sodium (40 mg/kg i.v.) and showing basal mean arterial blood pressure below 150 mm (Hg) were categorised as normotensive and above 150 mm Hg as hypertensive. Arterial blood pressure (BP) was recorded from one of the carotid artery through a stathum P23 DC pressure transducer and 7P1 low level DC preamplifier on a Grass Model P7 Polygraph, Signals from 7P1 preamplifier were used to trigger 7P4 Tachograph preamplifier for recording the heart rate (HR). Right femoral vein and Trachea were cannulated for intravenous injections and artificial ventilation respectively. Control responses to intravenous injection of noradrenaline (2-4 ug); acetyl choline (1-2 ug); histamine (102 ug) and isoprenaline (1-2 ug) were taken before and after the administration of test doses of each compounds. All the compounds were tested at fixed doses of 2.0 and 10 uM/kg i.v. Significant results are given in Table 2.
(b) Antihypertensive activity was observed in naturally hypertensive cats, rat abdominal aorta coarctation model of hypertension (Liu, J; Bishop, S.P. & Overbeck, H.W. Morphometric evidence for non pressure related arterial wall thickening in hypertension Circ. Res. 62, 1001-1010, 1988) and L-NAME (No synthase inhibition) induced hypertensive cats.

### Example 16

### Cardioprotective/antiischemic activity

The rats were killed by decapitation, heart were rapidly excised and placed in ice-cold HEPES tyrode buffer. Then isolated hearts were perfused retrogradely through coronary arteries using the Langendorff technique. The perfusion buffer consisted (in mM) NaCl 137, KCl 5.4, CaCl₂ 1.8, MgCl₂ 1.0, glucose 11.2 and HEPES 3.0. The buffer (pH 7.4) was continuously gassed with oxygen and maintained at 37°C. Coronary flow rate was maintained at 10 ml/min. The heart contracted spontaneously.

The perfused heart was allowed to equilibrate for 30 min. before initiation of any insult protocol. The text compounds were given at the time of reperfusion. Some of the compounds were dissolved in ethanol. Final concentration of ethanol in the perfusion buffer was 0.0001% and had no effect of its own on the parameter used.
(a) For brief period of ischemic insult (Hideo et al.). Pathophysiology and pathogenesis of stunned myocardium. J. Clin. Invest., 79, 950-961, 1987). Ischemia was initiated by stopping the flow for 16 min. followed by 30 min. reperfusion period. The durations were decided on the initial pilot experiments leading to 50% recovery of function.
(b) For prolong period of ischemic insult (Becker, L.C. & Ambrosio, G. Myocardial consequences of reperfusion. Prog. Cardiovas. Dis., 30, 23-44, 1987). Ischemia was initiated by stopping the flow for 30 min. followed by 30 min. reperfusion.

## Claims

1. 1-[4-Arylpiperazin-1-yl]-3-[2-oxopyrrolidin/piperidin-1-yl] propanes having general formula 1 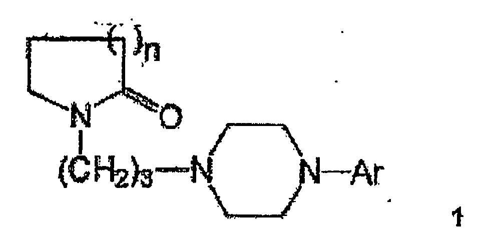 wherein Ar represents pyridyl, or a phenyl ring substituted with trifluoromethyl, halogen, alkyl, or alkoxy, and n = 1 or n = 2.

2. Compounds of formula 1 according to claim 1, wherein the preferred compounds are represented as following:
(a) 1-[4-(3-chlorophenyl)piperazin-1-yl]-3-[2-oxopyrrolidin-1-yl] propane.
(b) 1-[4-(4-chlorophenyl)piperazin-1-yl]-3-[2-oxopyrrolidin-1-yl] propane.
(c) 1-[4-(3-fluorophenyl)piperazin-1-yl]-3-[2-oxopyrrolidin-1-yl] propane.
(d) 1-[4-(4-fluorophenyl)piperazin-1-yl]-3-[2-oxopyrrolidin-1-yl] propane.
(e) 1-[4-(4-ethylphenyl)piperazin-1-yl]-3-[2-oxopyrrolidin-1-yl] propane.
(f) 1-[4-(2-ethylphenyl)piperazin-1-yl]-3-[2-oxopyrrolidin-1-yl] propane.
(g) 1-[4-(4-chlorophenyl)piperazin-1-yl]-3-[-oxopiperidin-1-yl] propane.
(h) 1-[4-(3-chlorophenyl)piperazin-1-yl]-3-[2-oxopiperidin-1-yl] propane.
(i) 1-[4-(4-fluorophenyl)piperazin-1-yl]-3-[2-oxopipeddin-1-yl] propane.
(j) 1-[4-(2-ethylphenyl)piperazin-1-yl]-3-[2-oxopiperidin-1-yl] propane.
(k) 1-[4-(2-methoxyphenyl) piperazin-1-yl]-3-[2-oxopiperidin-1-yl] propane.
(l) 1-[4-(2-pyridyl) piperazin-1-yl]-3-[2-oxopiperidin-1-yl] propane.
(m) 1-[4-(3-trifluoromethylphenyl)piperazin-1-yl]-3-[2-oxopiperidin-1-yl] propane.

3. A process for the synthesis of compounds of formula 1 according to claims 1 and 2, comprising condensing a pyridyl piperazine or an appropriately substituted phenylpiperazine of formula 4 where Ar represents pyridyl, or a phenyl ring substituted by trifluoromethyl, halogen, alkoxy or alkyl, with 1-chloro-3-[2-oxopyrrolidin-1-yl] propane of formula 3 (n=1) or 1-chloro-3-[2-oxopiperidin-1-yl] propane of formula 3 (n=2) in the presence of a base, a catalyst and an organic solvent at a temperature ranging from 70-120°C for a period varying between 8-14 hrs to produce the corresponding 1-[4-substituted arylpiperazin-1-yl]-3-[2-oxopyrrolidin-1-yl] propanes of formula 1(n=1) or 1-[4-substituted arylpiperazin-1-yl]-3-[2-oxopiperidin-1-yl] propanes of formula 1 (n=2) respectively.

4. A process according to claim 3, wherein the process for the synthesis of the intermediate 1-chloro-3-[2-oxopyrrolidin-1-yl] propane of formula 3 (n=1) and 1-chloro-3-[2-oxopiperidin-1-yl] propane of formula 3 (n=2) comprises reacting 2-pyrrolidone of formula 2 (n=1) or 2-piperidone of formula 2 (n=2) with 1-bromo-3-chloropropane in the presence of an aromatic solvents selected from xylene, toluene and a base selected from pulverised sodium or potassium metal or potassium *tert*. butoxide at a temperature in the range of 110-150°C for a period ranging from 80 minutes to 14 hrs.

5. A process according to claim 3, wherein the base used is selected from sodium carbonate or potassium carbonate.

6. A process according to claim 3, wherein the catalyst used is selected from sodium iodide or potassium iodide.

7. A process according to claim 3, wherein the molar ratio of the compounds of formula 3 and 4 is 1:1.

8. A process according to claim 3, wherein the solvent used is selected from DMF, toluene, xylene and the amount of the solvent used may range from 0.8-2.4 ml per mmol of the reacting compounds **3** and 4 of formula 3.

9. A process according to claim 3, wherein the molar ratio of the base to the compounds of formula 3 and 4 is 1:2.

10. A pharmaceutical composition comprising a compound of formula 1 according to claim 1 or 2, in admixture with a pharmaceutically acceptable carrier.

11. A process for the preparation of a pharmaceutical composition which comprises bringing a compound of the formula 1 according to claim 1 or 2 into association with a pharmaceutically acceptable additives.

12. The use of a compound according to claim 1 or 2, in the manufacture of a medicament for treating hypertension in mammals.

13. The use of a compound according to claim 1 or 2, in the manufacture of a medicament for treating peripheral vascular diseases in mammals.

14. The use of a compound according to claim 1 or 2, in the manufacture of a medicament for antagonising peripheral alpha-adrenergic receptor in mammals.

15. The use of a compound according to claim 1 or 2, in the manufacture of a medicament for treating diseases arising out of alterations/impairment in central/peripheral circulations and adrenergic receptors systems, such as myocardial ischemia, myocardial infarction (MI), angina pectoris, any cardiac surgical interventions, renal ischemia, circulatory insufficiency in extremities, stroke and trauma.

16. The use of a compound according to claim 1 or 2, in the manufacture of a medicament for treating reperfusion injury in mammals.

17. The use of a compound according to claim 1 or 2 in the manufacture of a medicament for treating ischemic diseases like myocardial infarction (MI), angina pectoris, any cardiac surgical interventions in mammals.

## Patentansprüche

1. 1-[4-Arylpiperazin-1-yl]-3-[2-oxopyrrolidin/piperidin-1-yl]propane der allgemeinen Formel 1 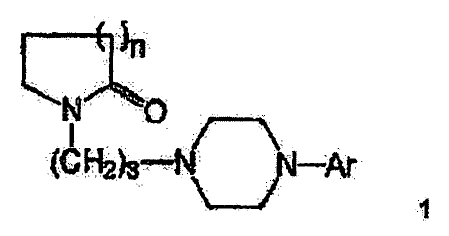 worin Ar Pyridyl oder einen mit Trifluormethyl, Halogen, Alkyl oder Alkoxy substituierten Phenylring bedeutet und n = 1 oder n = 2 ist.

2. Verbindungen der Formel 1 gemäß Anspruch 1, worin die bevorzugten Verbindungen wie folgt wiedergegeben sind:
(a) 1-[4-(3-Chlorphenyl)piperazin-1-yl]-3-[2-oxopyrrolidin-1-yl]propan,
(b) 1-[4-(4-Chlorphenyl)piperazin-1-yl]-3-[2-oxopyrrolidin-1-yl]propan,
(c) 1-[4-(3-Fluorphenyl)piperazin-1-yl]-3-[2-oxopyrrolidin-1-yl]propan,
(d) 1-[4-(4-Fluorphenyl)piperazin-1-yl]-3-[2-oxopyrrolidin-1-yl]propan,
(e) 1-[4-(4-Ethylphenyl)piperazin-1-yl]-3-[2-oxopyrrolidin-1-yl]propan,
(f) 1-[4-(2-Ethylphenyl)piperazin-1-yl]-3-[2-oxopyrrolidin-1-yl]propan,
(g) 1-[4-(4-Chlorphenyl)piperazin-1-yl]-3-[-oxopiperidin-1-yl]propan,
(h) 1-[4-(3-Chlorphenyl)piperazin-1-yl]-3-[2-oxopiperidin-1-yl]propan,
(i) 1-[4-(4-Fluorphenyl)piperazin-1-yl]-3-[2-oxopiperidin-1-yl]propan,
(j) 1-[4-(2-Ethylphenyl)piperazin-1-yl]-3-[2-oxopiperidin-1-yl]propan,
(k) 1-[4-(2-Methoxyphenyl)piperazin-1-yl]-3-[2-oxopiperidin-1-yl]propan,
(l) 1-[4-(2-Pyridyl)piperazin-1-yl]-3-[2-oxopiperidin-1-yl]propan und
(m) 1-[4-(3-Trifluormethylphenyl)piperazin-1-yl]-3-[2-oxopiperidin-1-yl]propan.

3. Verfahren zur Synthese von Verbindungen der Formel 1 gemäß den Ansprüchen 1 und 2, umfassend das Kondensieren eines Pyridylpiperazins oder eines geeignet substituierten Phenylpiperazins der Formel 4, worin Ar Pyridyl oder einen mit Trifluorphenyl, Halogen, Alkoxy oder Alkyl substituierten Phenylring bedeutet, mit 1-Chlor-3-[2-oxopyrrolidin-1-yl]propan der Formel 3 (n = 1) oder 1-Chlor-3-[2-oxo-piperidin-1-yl]propan der Formel 3 (n = 2) in Gegenwart einer Base, eines Katalysators und eines organischen Lösemittels bei einer Temperatur in dem Bereich von 70 bis 120°C für eine Zeit zwischen 8 bis 14 Stunden zum Erhalt der entsprechend 1-[4-substituierten Arylpiperazin-1-yl]-3-[2-oxopyrrolidin-1-yl]propane der Formel 1 (n = 1) oder 1-[4-substituierten Arylpiperazin-1-yl]-3-[2-oxopiperidin-1-yl]propane der Formel 1 (n = 2).

4. Verfahren gemäß Anspruch 3, worin das Verfahren zur Synthese der Zwischenprodukte 1-Chlor-3-[2-oxopyrrolidin-1-yl]propan der Formel 3 (n = 1) und 1-Chlor-3-[2-oxopiperidin-1-yl]propan der Formel 3 (n = 2) umfasst das Umsetzen von 2-Pyrrolidon der Formel 2 (n = 1) oder 2-Piperidon der Formel 2 (n = 2) mit 1-Brom-3-chlorpropan in Gegenwart eines aromatischen Lösemittels, ausgewählt aus Xylol und Toluol, und einer Base, ausgewählt aus pulverisiertem Natrium- oder Kaliummetall oder Kalium-tert-butoxid, bei einer Temperatur in dem Bereich von 110 bis 150°C für eine Zeit in dem Bereich von 80 Minuten bis 14 Stunden.

5. Verfahren gemäß Anspruch 3, worin die Base aus Natriumcarbonat oder Kaliumcarbonat ausgewählt ist.

6. Verfahren gemäß Anspruch 3, worin der verwendete Katalysator aus Natriumiodid oder Kaliumiodid ausgewählt ist.

7. Verfahren gemäß Anspruch 3, worin das molare Verhältnis der Verbindungen der Formel 3 und 4 1 : 1 beträgt.

8. Verfahren gemäß Anspruch 3, worin das Lösemittel aus DMF, Toluol und Xylol ausgewählt ist und die Menge des verwendeten Lösemittels in dem Bereich von 0,8 bis 2,4 ml pro mmol der reagierenden Verbindungen 3 und 4 der Formel 3 liegen kann.

9. Verfahren gemäß Anspruch 3, worin das molare Verhältnis der Base zu den Verbindungen der Formel 3 1 : 2 beträgt.

10. Pharmazeutische Zusammensetzung, umfassend eine Verbindung der Formel 1 gemäß Anspruch 1 oder 2 in Mischung mit einem pharmazeutisch annehmbaren Träger.

11. Verfahren zur Herstellung einer pharmazeutischen Zusammensetzung, welche das Inkontaktbringen einer Verbindung der Formel 1 gemäß Anspruch 1 oder 2 mit pharmazeutisch annehmbaren Zusätzen umfasst.

12. Verwendung einer Verbindung gemäß Anspruch 1 oder 2 bei der Herstellung eines Medikaments zur Behandlung von Bluthochdruck in Säugern.

13. Verwendung einer Verbindung gemäß Anspruch 1 oder 2 bei der Herstellung eines Medikaments zur Behandlung von peripheren Gefäßerkrankungen in Säugern.

14. Verwendung einer Verbindung gemäß Anspruch 1 oder 2 bei der Herstellung eines Medikaments zum Antagonisieren peripherer α-adrenerger Rezeptoren in Säugern.

15. Verwendung einer Verbindung gemäß Anspruch 1 oder 2 bei der Herstellung eines Medikaments zur Behandlung von Krankheiten, die von Änderungen/Verschlechterung in zentralen/peripheren Kreisläufen und adrenergen Rezeptorsystemen herrühren, wie myokardiale Ischämie, myokardialer Infarkt (MI), Angina pectoris, kardiale chirurgische Eingriffe, renale Ischämie, Kreislaufinsuffizienz in Extremitäten, Schlaganfall und Trauma.

16. Verwendung einer Verbindung gemäß Anspruch 1 oder 2 bei der Herstellung eines Medikaments zur Behandlung eines Reperfusionsschadens in Säugern.

17. Verwendung einer Verbindung gemäß Anspruch 1 oder 2 bei der Herstellung eines Medikaments zur Behandlung ischämischer Erkrankungen, wie myokardialer Infarkt (MI), Angina pectoris und kardiale chirurgische Eingriffe in Säugern.

## Revendications

1. 1-[4-Arylpipérazin-1-yl]-3-[2-oxopyrrolidin/pipéridin-1-yl] propanes répondant à la formule générale 1 : 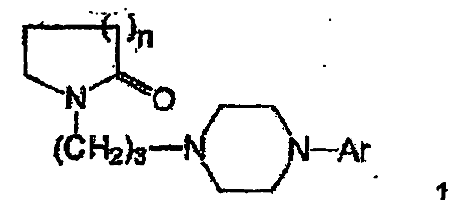 dans laquelle Ar représente un pyridyle, ou un noyau phényle substitué par du trifluorométhyle, un halogène, un alkyle, ou un alcoxy, et n = 1 ou n = 2.

2. Composés de formule 1 selon la revendication 1, dans lesquels les composés préférés sont représentés comme suit :
(a) 1-[4-(3-chlorophényl) pipérazin-1-yl]-3-[2-oxopyrrolidin-1-yl] propane.
(b) 1-[4-(4-chlorophényl) pipérazin-1-yl]-3-[2-oxopyrrolidin-1-yl] propane.
(c) 1-[4-(3-fluorophényl) pipérazin-1-yl]-3-[2-oxopyrrolidin-1-yl] propane.
(d) 1-[4-(4-fluorophényl) pipérazin-1-yl]-3-[2-oxopyrrolidin-1-yl] propane.
(e) 1-[4-(4-éthylphényl) pipérazin-1-yl]-3-[2-oxopyrrolidin-1-yl] propane.
(f) 1-[4-(2-éthylphényl) pipérazin-1-yl]-3-[2-oxopyrrolidin-1-yl] propane.
(g) 1-[4-(4-chlorophényl) pipérazin-1-yl]-3-[oxopipéridin-1-yl] propane.
(h) 1-[4-(3-chlorophényl)pipérazin-1-yl]-3-[2-oxopipéridin-1-yl] propane.
(i) 1-[4-(4-fluorophényl)pipérazin-1-yl]-3-[2-oxopipéridin-1-yl] propane.
(j) 1-[4-(2-éthylphényl) pipérazin-1-yl]-3-[2-oxopipéridin-1-yl] propane.
(k) 1-[4-(2-méthoxyphényl) pipérazin-1-yl]-3-[2-oxopipéridin-1-yl] propane.
(l) 1-[4-(2-pyridyl) pipérazin-1-yl]-3-[2-oxopipéridin-1-yl] propane.
(m) 1-[4-(3-trifluorométhylphényl) pipérazin-1-yl]-3-[2-oxopipéridin-1-yl] propane.

3. Procédé de synthèse des composés de formule 1 selon les revendications 1 et 2, comprenant la condensation d'une pyridyl-pipérazine ou d'une phénylpipérazine substituée de manière appropriée de formule 4 dans laquelle Ar représente un pyridyle, ou un noyau phényle substitué par du trifluorométhyle, un halogène, un alcoxy, ou un alkyle, avec le 1-chloro-3-[2-oxopyrrolidin-1-yl) propane de formule 3 (n = 1) ou le 1-chloro-3-[2-oxopipéridin-1-yl] propane de formule 3 (n = 2) en présence d'une base, d'un catalyseur et d'un solvant organique à une température comprise dans la plage de 70 à 120°C pendant une période de temps comprise dans la plage de 8 à 14 heures pour produire les 1-[aryl(substitué en position 4)-pipérazin-1-yl]-3-[2-oxopyrrolidin-1-yl] propanes correspondants de formule 1 (n = 1) ou les 1-[aryl(substitué en position 4)-pipérazin-1-yl]-3-[2-oxopipéridin-1-yl] propanes correspondants de formule 1 (n = 2), respectivement.

4. Procédé selon la revendication 3, dans lequel le procédé de synthèse des intermédiaires 1-chloro-3-[2-oxopyrrolidin-1-yl] propane de formule 3 (n = 1) et 1-chloro-3-[2-oxopipéridin-1-yl] propane de formule 3 (n = 2) comprend la mise en réaction d'une 2-pyrrolidone de formule 2 (n = 1) ou d'une 2-pipéridone de formule 2 (n = 2) avec le 1-bromo-3-chloropropane en présence d'un solvant aromatique choisi parmi le xylène, le toluène et d'une base choisie parmi le sodium ou le potassium métal en poudre ou le tert-butylate de potassium à une température comprise dans la plage de 110 à 150°C pendant une période de temps comprise dans la plage de 80 minutes à 14 heures.

5. Procédé selon la revendication 3, dans lequel la base utilisée est choisie parmi le carbonate de sodium ou le carbonate de potassium.

6. Procédé selon la revendication 3, dans lequel le catalyseur utilisé est choisi parmi l'iodure de sodium ou l'iodure de potassium.

7. Procédé selon la revendication 3, dans lequel le rapport molaire des composés de formule 3 et 4 est de 1:1.

8. Procédé selon la revendication 3, dans lequel le solvant utilisé est choisi parmi le DMF, le toluène, le xylène et la quantité de solvant utilisée peut être comprise dans la plage de 0,8 à 2,4 ml par mmol de réactifs 3 et 4 de formule 3.

9. Procédé selon la revendication 3, dans lequel le rapport molaire de la base aux composés de formule 3 et 4 est de 1:2.

10. Composition pharmaceutique comprenant un composé de formule 1 selon la revendication 1 ou 2, en mélange avec un véhicule acceptable du point de vue pharmaceutique.

11. Procédé de préparation d'une composition pharmaceutique qui comprend l'apport d'un composé de formule 1 selon la revendication 1 ou 2 en association avec des additifs acceptables du point de vue pharmaceutique.

12. Utilisation d'un composé selon la revendication 1 ou 2, pour la fabrication d'un médicament pour le traitement de l'hypertension chez les mammifères.

13. Utilisation d'un composé selon la revendication 1 ou 2, pour la fabrication d'un médicament pour le traitement des maladies vasculaires périphériques chez les mammifères.

14. Utilisation d'un composé selon la revendication 1 ou 2, pour la fabrication d'un médicament pour antagoniser le récepteur alpha adrénergique périférique chez les mammifères.

15. Utilisation d'un composé selon la revendication 1 ou 2, pour la fabrication d'un médicament pour le traitement de maladies issues de modifications/dysfonctionnement au niveau de la circulation centrale/périphérique et des systèmes de récepteurs adrénergiques, telles que l'ischémie du myocarde, l'infarctus du myocarde (MI), l'angine de poitrine, de quelconques interventions chirurgicales cardiaques, l'ischémie rénale, l'insuffisance circulatoire dans les membres, une attaque et un traumatisme.

16. Utilisation d'un composé selon la revendication 1 ou 2, pour la fabrication d'un médicament pour le traitement de lésions de reperfusion chez les mammifères.

17. Utilisation d'un composé selon la revendication 1 ou 2, pour la fabrication d'un médicament pour le traitement de maladies ischémiques telles que l'infarctus du myocarde (MI), l'angine de poitrine, de quelconques interventions chirurgicales cardiaques chez les mammifères.
